(19) Europäisches Patentamt
European Patent Office
Office européen des brevets

(11) Numéro de publication : **0 102 858**
**B1**

(12) **FASCICULE DE BREVET EUROPÉEN**

(45) Date de publication du fascicule du brevet : **30.12.86**

(51) Int. Cl.⁴ : **C 07 C 21/06**, C 07 C 17/26

(21) Numéro de dépôt : **83401315.3**

(22) Date de dépôt : **24.06.83**

(54) **Procédé de fabrication de chlorure de vinyle.**

(30) Priorité : **06.07.82 FR 8211810**

(43) Date de publication de la demande : **14.03.84 Bulletin 84/11**

(45) Mention de la délivrance du brevet : **30.12.86 Bulletin 86/52**

(84) Etats contractants désignés : **BE DE FR GB IT NL SE**

(56) Documents cités :
**EP-A- 0 084 564**
**FR-A- 1 275 347**
**FR-A- 2 248 254**
**US-A- 4 373 109**

(73) Titulaire : **ATOCHEM**
**4 & 8, Cours Michelet La Défense 10**
**F-92800 Puteaux (FR)**

(72) Inventeur : **Olah, George Andrew**
**2252 Gloaming Way**
**Beverly Hills California 90210 (US)**

**EP 0 102 858 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé de fabrication de chlorure de vinyle à partir de chlorure de méthyle.

Le chlorure de vinyle dont la production s'est très fortement développée au cours des trente dernières années sert essentiellement, en tant que monomère, comme produit de base à la fabrication des résines vinyliques chlorées.

A l'origine, le chlorure de vinyle monomère était fabriqué par addition d'acide chlorhydrique sur l'acétylène. Ce procédé est maintenant remplacé par la chloration ou l'oxychloration de l'éthylène avec deshydrochloration thermique des produits obtenus. Ces procédés sont largement décrits dans la littérature comme par exemple dans « Industrial Organic Chemistry » K. WEISSERMEL and H.J. ARPE, Verlag Chemie, Weinheim, New-York 1978.

Les procédés actuels de fabrication, basés sur l'éthylène, sont dépendants des sources pétrochimiques en particulier du naphta ou des produits de cracking des hydrocarbures légers.

Le procédé selon l'invention permet de fabriquer le chlorure de vinyle par condensation du chlorure de méthyle obtenu à partir de méthane ou de méthanol, et est caractérisé en ce que la condensation du chlorure de méthyle est effectuée en présence d'un catalyseur choisi parmi les oxydes, oxyhalogénures ou sulfures des métaux de transition des groupes IV, V, VI et VII de la table périodique, ou encore parmi les alumino-silicates, et est suivie d'une oxychloration des produits de condensation, cette oxychloration étant elle-même suivie d'une deshydrochloration connue en soi.

Le procédé peut globalement se schématiser en trois étapes de la façon suivante :

1. $2CH_3Cl \longrightarrow$ produit de condensation + HCl

2. Produits de condensation + HCl + 1/2 $O_2$

$\xrightarrow{\text{oxychloration}} ClCH_2CH_2Cl + H_2O$

3. $ClCH_2CH_2Cl \xrightarrow{\text{deshydrochloration}} CH_2 = CHCl + HCl$

L'acide chlorhydrique de la troisième étape peut être récupéré et recyclé à l'oxychloration des produits de condensation ou à la chloration du méthanol en chlorure de méthyle :

$$CH_3OH + HCl \longrightarrow CH_3Cl + H_2O$$

Une autre possibilité de récupération du HCl résiduaire peut être retenue, à savoir : l'oxychloration du méthane :

$$CH_4 + HCl + 1/2\ O_2 \longrightarrow CH_3Cl + H_2O$$

Suivant la nature de la matière première utilisée, la réaction globale peut être symbolisée par une des deux équations :

$$2\ CH_3OH + HCl + 1/2\ O_2 \Longrightarrow CH_2 = CHCl + 3\ H_2O$$

$$4\ CH_4 + Cl_2 + 2{,}5\ O_2 \Longrightarrow 2\ CH_2 = CHCl + 5\ H_2O$$

La condensation selon la première étape est une réaction chimique connue et décrite dans le brevet des Etats-Unis d'Amérique 4 373 109, correspondant à la demande européenne numéro 82902664.0 publiée sous le numéro EP-A-0084864, mais les résultats particulièrement satisfaisants, dus à la diminution des réactions secondaires, sont obtenus lorsqu'elle est effectuée en présence de catalyseur bifonctionnel acide-base, à une température de 250 à 450 °C et de préférence comprise entre 325 et 375 °C avec une vitesse spatiale horaire des gaz (GHSV) de 50 à 300 et de préférence de 75 à 100. Les catalyseurs bifonctionnels recommandés dérivent des chalconides — oxydes, oxyhalogénures ou sulfures — des métaux de transition des groupes IV, V, VI, et VII de la table périodique et particulièrement des chalconides du tantale, niobium, zirconium, tungstène, titane, chrome. Ils sont habituellement déposés sur un support tel que l'alumine, zircone, silice et autres. On peut encore utiliser comme catalyseurs les aluminosilicates comme la forme acide d'une mordenite ayant un rapport Si/Al d'au moins 10. La condensation peut également s'effectuer en présence d'un diluant inerte, tel que, par exemple, un excès de méthane ou de l'azote.

La réaction d'oxychloration s'effectue habituellement en présence de chlorure de cuivre comme catalyseur à des températures comprises entre 200 et 300 °C et plus particulièrement voisines de 250 °C

sous une pression de 1 à 10 bars.

La réaction de deshydrochloration est purement thermique. Le dichloréthane gazeux est transformé en chlorure de vinyle par passage dans un réacteur à une température de 480 à 530 °C sous une pression de 5 à 20 bars. En général, environ 50 % du dichloréthane est transformé en chlorure de vinyle, le dichloréthane n'ayant pas réagi étant recyclé.

Le chlorure de méthyle peut provenir de la chloration du méthanol ou du méthane.

Un procédé intéressant d'obtention du chlorure de méthyle consiste à faire réagir sur le méthanol de l'acide chlordhydrique, et plus particulièrement l'acide chlorhydrique formé en sous produit de la troisième étape décrite ci-dessus. La réaction peut s'effectuer en phase liquide, entre 180 et 240 °C, en présence d'un catalyseur à base habituellement de $ZnCl_2$ sous une pression de 1 à 5 bars. Le rapport molaire $HCl/CH_3OH$ est de préférence compris entre 1,1 et 1,3. Cette réaction peut également s'effectuer en phase vapeur à une température comprise entre 400 et 450 °C en présence d'alumine comme catalyseur.

Un tel procédé permet de fabriquer directement en continu le chlorure de vinyle à partir de méthanol en passant par le stade intermédiaire du chlorure de méthyle selon les schémas :

$$2\ CH_3OH + 2\ HCl \longrightarrow 2\ CH_3Cl + 2\ H_2O$$

$$2\ CH_3Cl \xrightarrow{\text{condensation puis } 1/2\ O_2} ClCH_2CH_2Cl + H_2O$$

$$ClCH_2CH_2Cl \longrightarrow CH_2 = CHCl + HCl$$

Un schéma voisin peut être envisagé à partir du méthane :

$$CH_4 + Cl \longrightarrow CH_3Cl + HCl$$

$$3\ CH_4 + 3\ HCl + 3/2\ O_2 \longrightarrow 3\ CH_3Cl + 3\ H_2O$$

$$4\ CH_3Cl \xrightarrow{\text{condensation puis } O_2} 2\ ClCH_2CH_2Cl + 2\ H_2O$$

$$2\ ClCH_2CH_2Cl \longrightarrow 2\ CH_2 = CHCl + 2\ HCl$$

La chloration du méthane peut s'effectuer à une température comprise entre 400 et 450 °C sous une légère pression.

Le chlorure de méthyle peut également être obtenu par photochimie. Ces procédés d'obtention du chlorure de méthyle sont par exemple, décrits par : F. ASINGER « Paraffins Chemistry and Technology » Pergamon Press, New-York, 1968. — M.L. POUTSMA « Methods in Free Radical Chemistry », Vol. II, E.S. Huyser, Ed., M. Dekker, New-York 1969 — R. WEISSERMEL and H.J. ARPE « Industrial Organic Chemistry » Verlag Chemie, 1978, pp. 46-47.

Un autre procédé consiste en une chloration ionique ou initiée par un métal noble du méthane effectuée à une température comprise entre 100 et 325 °C et plus particulièrement entre 200° et 300 °C en présence d'un catalyseur choisi parmi les halogénures ou oxyhalogénures des métaux des groupes IV, V, VI de la table périodique, comme par exemple le tantale, le niobium, le zirconium, le tungstène, le titane, le chrome et autres, ou encore parmi les catalyseurs métalliques du groupe VIII, tel que le platine ou le palladium. Ce type de catalyseur est de préférence déposé sur un support approprié tel que l'alumine ou le graphite. On peut encore utiliser les catalyseurs du type Bronstead tel que les acides sulfoniques perfluorés ou les résines acides sulfoniques. Cette chloration ionique ou initiée par un métal noble est illustrée dans la demande de brevet américain 298 390.

De façon générale, le rapport molaire $Cl_2/CH_4$ est habituellement compris entre 1 et 5 et plus précisément entre 1,5 et 3.

Les exemples suivants servent à illustrer l'invention, les compositions données étant étalonnées, même s'il n'en est pas fait état, pour fournir un taux de conversion total de 100 %.

## Exemple 1

Dans un rapport respectif de 3/1 du méthane et du chlorure passent dans un réacteur tubulaire à une température de 235 °C sur un catalyseur composé de 10 % en poids d'oxyfluorure de tantale déposé sur 90 % en poids d'alumine. Le GHSV est d'environ 50.

Le produit de réaction contenant, outre l'excès de méthane, 96,5 % de chlorure de méthyle et 3,5 % de chlorure d'éthyle ainsi que l'acide chlorhydrique correspondant formé est, sans séparation, passé à travers un second réacteur sur un catalyseur composé de 20 % d'oxyde de tungstène supporté par 80 % d'alumine. Le GHSV est d'environ 80, le mélange gazeux passant sur le catalyseur à la température de 365 °C.

Du produit de l'étape de condensation, on extrait le méthane ainsi que le chlorure de méthyle non réagi, qui est recyclé. La fraction contenant le produit de condensation y compris l'acide chlorhydrique anhydre est mise à réagir avec l'oxygène en présence de $CuCl_2$ comme catalyseur sous 2 à 3 atmosphères à une température de 220°-240 °C. Le 1,2 dichloréthane (EDC) obtenu avec un rendement de 95 % et une sélectivité de 98 % est deshydrochloré thermiquement à 500 °C.

On obtient le chlorure de vinyle avec 98 % de sélectivité basée sur le dichloréthane. L'acide chlorhydrique formé en sous produit est recyclé via oxychloration.

Exemple 2

On fait réagir à 320-370 °C en quantité stœchiométrique l'alcool méthylique et l'acide chlorhydrique en présence d'alumine comme catalyseur. Le produit de réaction contenant le chlorure de méthyle, sans séparation, est directement envoyé dans un réacteur de condensation, les conditions de condensation, d'oxychloration et de deshydrochloration restant identiques à celles de l'exemple 1.

Exemple 3

On opère dans les conditions de l'exemple 1, le catalyseur à base d'oxyfluorure de tantale étant remplacé par 0,5 % de palladium déposé sur 99,5 % d'alumine et le catalyseur à base d'oxyde de tungstène de l'étape de condensation étant remplacé par la forme acide de mordenite. On obtient le chlorure de vinyle avec 99 % de sélectivité basée sur le dichloréthane.

**Revendications**

1. Procédé de fabrication de chlorure de vinyle par condensation du chlorure de méthyle obtenu à partir de méthane ou de méthanol, caractérisé en ce que la condensation du chlorure de méthyle est effectuée en présence d'un catalyseur choisi parmi les oxydes, oxyhalogénures ou sulfures des métaux de transition des groupes IV, V, VI, et VII de la table périodique, ou encore parmi les aluminosilicates, et est suivie d'une oxychloration des produits de condensation, cette oxychloration étant elle-même suivie d'une deshydrochloration connue en soi.
2. Procédé selon la revendication 1 caractérisé en ce que l'acide chlorhydrique formé à la deshydrochloration est recyclé à l'oxychloration des produits de condensation.
3. Procédé selon la revendication 1 caractérisé en ce que l'acide chlorhydrique formé à la deshydrochloration est recyclé à la chloration du méthanol en chlorure de méthyle.
4. Procédé selon la revendication 1 caractérisé en ce que l'acide chlorhydrique formé à la deshydrochloration est recyclé à l'oxychloration du méthane en chlorure de méthyle.

**Claims**

1. Process for manufacturing vinyl chloride by condensation of methyl chloride obtained from methane or methanol, characterized in that the condensation of the methyl chloride is performed in the presence of a catalyst chosen from oxides, oxyhalides or sulphides of transition metals of Groups IV, V, VI and VII of the Periodic Table, or alternatively from aluminosilicates, and is followed by oxychlorination of the condensation products, this oxychlorination itself being followed by a dehydrochlorination known per se.
2. Process according to Claim 1, characterized in that the hydrochloric acid formed in the dehydrochlorination is recycled to the oxychlorination of the condensation products.
3. Process according to Claim 1, characterized in that the hydrochloric acid formed in the dehydrochlorination is recycled to the chlorination of methanol to methyl chloride.
4. Process according to Claim 1, characterized in that the hydrochloric acid formed in the dehydrochlorination is recycled to the oxychlorination of methane to methyl chloride.

**Patentansprüche**

1. Verfahren zur Herstellung von Vinylchlorid durch Kondensation von Methylchlorid, erhalten ausgehend von Methan oder Methanol, dadurch gekennzeichnet, daß die Kondensation von Methylchlorid in Gegenwart eines Katalysators durchgeführt wird, der ausgewählt wird aus den Oxiden, Oxyhalogeniden oder Sulfiden der Übergangsmetalle der Gruppen IV, V, VI und VII des Periodensystems, oder auch aus den Aluminiumsilicaten, und gefolgt wird von einer Oxychlorierung der Kondensationsprodukte und diese Oxychlorierung ihrerseits von einer an sich bekannten Dehydrochlorierung gefolgt wird.
2. Verfahren nach Anspruch 1, dadurch gekennzeichnet, daß die bei der Dehydrochlorierung entstandene Chlorwasserstoffsäure in die (Stufe der) Oxychlorierung der Kondensationsprodukte

zurückgeführt wird.

3. Verfahren nach Anspruch 1, dadurch gekennzeichnet, daß die bei der Dehydrochlorierung entstandene Chlorwasserstoffsäure in die (Stufe der) Chlorierung von Methanol zu Methylchlorid zurückgeführt wird.

4. Verfahren nach Anspruch 1, dadurch gekennzeichnet, daß die bei der Dehydrochlorierung entstandene Chlorwasserstoffsäure in die (Stufe der) Oxychlorierung von Methan zu Methylchlorid zurückgeführt wird.